# FASCICULE DE BREVET EUROPEEN

(11) **EP 2 999 687 B1**
(45) Date de publication et mention de la délivrance du brevet: **11.03.2020**
(21) Numéro de dépôt: 14725160.7
(22) Date de dépôt: 19.05.2014
(51) Int. Cl.: C07C 51/43, C07C 51/47, C07C 51/48, C07C 51/00, C07C 59/64

(54) **PROCEDE OPTIMISE D'EXTRACTION D'ACIDE FERULIQUE AVEC PRETRAITEMENT**
OPTIMIERTES VERFAHREN ZUR EXTRAKTION VON FERULASÄURE UND VORBEHANDLUNG
OPTIMIZED PROCESS FOR EXTRACTION OF FERULIC ACID WITH PRETREATMENT

(30) Priorité: 21.05.2013 FR 1354533; 05.09.2013 FR 1302058
(43) Date de publication de la demande: 30.03.2016
(73) Titulaire: Rhodia Operations, 75009 Paris (FR)
(72) Inventeur: REVELANT, Denis, 69740 Genas (FR); FOUCHER, Stéphanie, 69330 Meyzieu (FR); HORBEZ, Dominique, 95130 Franconville (FR); MARION, Philippe, 69390 Vernaison (FR)
(74) Mandataire: Menville, Laure
(86) Numéro de dépôt international: PCT/EP2014/060259
(87) Numéro de publication internationale: WO 2014/187784

(56) Documents cités:
- EP-A1- 0 885 968
- WO-A1-2004/110975

## Description

La présente invention se rapporte au domaine de la récupération de l'acide férulique naturel présent dans un effluent aqueux obtenu par traitement de matière végétale en vue de l'extraire avec une haute pureté afin de pouvoir l'employer comme matière première dans différents types d'industrie, en particulier l'industrie chimique, cosmétique, pharmaceutique, biotechnologique ou agro-alimentaire. La présente invention trouve très avantageusement une application pour la préparation d'arômes à partir d'acide férulique purifié, en particulier d'arômes alimentaires, notamment la vanilline.

### Etat de la technique antérieure

L'acide férulique est présent dans de nombreuses graines comme le riz, le blé, le maïs ou encore l'avoine. On le trouve également dans des sous-produits de l'industrie agro-alimentaire tels que les drêches de maïs (amidonnerie), la bagasse (transformation de la canne à sucre), la pulpe de betterave ou le résidu de raffinage de l'huile de son de riz, appelé « soap stock ». Il est connu de récupérer l'acide férulique par extraction du son de blé, de riz ou de maïs après cuisson alcaline ou traitement enzymatique. Après séparation de la matière végétale solide résiduelle, on obtient un flux aqueux dilué formé de 80 à 99% poids d'eau et également d'acide férulique, de polysaccharides et de sels minéraux. Le brevet US 5.288.902 décrit un procédé de cuisson alcaline du résidu résultant du raffinage de l'huile de son de riz. L'acide férulique est purifié par recristallisation après extraction des impuretés à l'hexane. La demande de brevet WO 2004/110975 décrit le traitement direct du jus de cuisson, à la chaux, du grain de maïs en procédant notamment à une étape d'acidification dudit effluent, appelé nejayote, issu de la cuisson alcaline puis à une étape d'adsorption de l'acide férulique sur une résine synthétique ou charbon activé suivie d'une élution avec un solvant organique et enfin d'une étape de recristallisation. La demande de brevet WO 2001/067891 décrit un prétraitement du son de riz par extrusion en présence d'eau suivi d'une hydrolyse enzymatique en présence de cellulase et/ou d'hémicellulase telles que les mannases, glucanase, arabinases. L'acide férulique est extrait à l'aide d'un solvant organique tandis que la fraction aqueuse est traitée avec une α-amylase pour générer une fraction de polysaccharides solubles.

De manière générale les procédés décrits conduisent à une consommation d'eau importante avec une valorisation très partielle des co-produits de traitement, en particulier la matière végétale résiduelle et les polysaccharides. L'impact environnemental n'est pas optimisé, contribuant ainsi à un appauvrissement des nappes phréatiques et à un accroissement de la charge organique rejetée dans l'environnement. De plus, la présence de polysaccharides pendant la phase de purification de l'acide férulique entraîne une perte de rendement significative et compromet la rentabilité économique du procédé.

Aussi pour remédier aux inconvénients rencontrés avec la mise en oeuvre des procédés antérieurs, la présente invention propose la mise en oeuvre d'un procédé propre d'extraction d'acide férulique conduisant à la production d'acide férulique avec une pureté améliorée et un rendement accru. L'acide férulique ainsi obtenu est avantageusement transformé en vanilline naturelle par un procédé de bio-fermentation.

### Description de l'invention

La présente invention a pour objet un procédé d'extraction d'acide férulique présent dans une phase aqueuse, obtenue par traitement d'au moins une matière végétale, et contenant également des polysaccharides, ledit procédé comprenant au moins les étapes suivantes :
1) le traitement de ladite matière végétale suivi d'une séparation solide/liquide pour récupérer une phase solide et une phase liquide aqueuse comprenant l'acide férulique et lesdits polysaccharides,
2) le traitement de ladite phase liquide pour séparer sélectivement les polysaccharides d'une part et l'acide férulique présent dans une fraction aqueuse d'autre part, par adsorption sélective des polysaccharides ou par ultrafiltration
3) éventuellement la concentration de ladite fraction aqueuse contenant l'acide férulique pour récupérer un flux concentré en acide férulique,
4) la récupération sous forme solide de l'acide férulique.

Conformément au procédé de l'invention, lorsque l'étape 3) est opérée, l'ordre des étapes 2) et 3) est indifférent une fois que le traitement selon ladite étape 1) a été effectué. De manière préférée, ladite étape 2) précède ladite étape 3). Quel que soit l'ordre des étapes 2) et 3), le flux qui est soumis à l'étape de récupération selon ladite étape 4) du procédé de l'invention est un flux concentré en acide férulique et sensiblement appauvri en polysaccharides, c'est-à-dire qu'il contient moins de 500 ppm, de préférence moins de 100 ppm de polysaccharides.

Conformément à l'invention, l'étape de séparation sélective des polysaccharides et l'étape de concentration de la fraction aqueuse contenant l'acide férulique sont des étapes de prétraitement de ladite phase aqueuse, obtenue à l'issue de ladite étape 1), mises en oeuvre avant de procéder à la récupération de l'acide férulique proprement dite selon ladite étape 4).

La matière végétale mise en œuvre dans le procédé de l'invention est avantageusement choisie parmi le son de blé, le son de riz, le son de maïs, le son d'avoine, les drêches de maïs, la bagasse, la pulpe de betterave et le résidu de raffinage de l'huile de son de riz et leurs mélanges. De manière avantageuse, ladite matière végétale est choisie parmi le son de blé, le son de riz, le son de maïs et les drêches de maïs.

Le traitement de ladite matière végétale selon ladite étape 1) du procédé de l'invention consiste à soumettre la matière végétale à une décoction alcaline et/ou à un traitement enzymatique de manière à libérer les espèces chimiques constituant ou reliées à la cellulose ou l'hémicellulose du végétal de départ. En particulier, le traitement selon ladite étape 1) libère l'acide férulique et les polysaccharides. De manière préférée, ledit traitement selon ladite étape 1) consiste au moins en la cuisson ou décoction alcaline d'au moins une des matières végétales brutes citées ci-dessus. Le traitement par décoction alcaline n'entraine aucune modification structurale des espèces chimiques initiales, il s'agit simplement d'une libération par hydrolyse de fonctions spécifiques, notamment des ponts esters. Au sens de la directive européenne (RÈGLEMENT (CE) N° 1334/2008 DU PARLEMENT EUROPÉEN ET DU CONSEIL du 16 décembre 2008), un tel traitement physique permet de conserver les critères de naturalité des composés libérés, à savoir l'acide férulique et les polysaccharides.

Le traitement par décoction alcaline (étape 1) consiste à faire macérer et cuire ladite matière végétale dans une solution alcaline ou une suspension alcaline. Le rapport massique matière végétale / solution alcaline est compris entre 0,05 et 0,5. La teneur en base dans la solution alcaline est comprise entre 1 et 30 % poids. La base utilisée pour ce traitement est avantageusement choisie parmi l'hydroxyde de sodium, l'hydroxyde de potassium et le carbonate de sodium. La température à laquelle on conduit la décoction alcaline est préférentiellement comprise entre 60 et 120°C. La durée opératoire de ce traitement est préférentiellement comprise entre 2 et 8 heures. La décoction alcaline est avantageusement réalisée à l'aide d'une cuve agitée munie d'un mobile d'agitation, d'une double enveloppe de chauffage et de contre-pales permettant d'optimiser les conditions de transfert de matière et d'énergie. La matière végétale brute est introduite dans ladite cuve contenant une solution alcaline diluée. En fin de décoction, le mélange obtenu est diphasique : la phase solide contient des fibres de cellulose et d'hémicellulose constitutives des parois des cellules végétales tandis que la phase liquide contient des polysaccharides dissous, des sucres élémentaires, des sels minéraux, des protéines et de l'acide férulique salifié.

De manière préférée, la qualité du transfert de matière dans l'étape de décoction alcaline est optimisée en utilisant des technologies à fort coefficient de cisaillement(γ 5000 s⁻¹) couplant transfert d'énergie efficace et mise en contact des constituants du mélange. Parmi ces technologies, on peut citer notamment les extrudeuses bi-vis et les broyeurs homogénéiseurs. Elles présentent l'avantage de minimiser les quantités de solvant et peuvent être opérées en étapes successives ou parallèles. Par exemple, dans l'extrudeuse bis-vis, on peut alimenter en continu la matière végétale à traiter et la solution alcaline en contrôlant finement les débits, température et agencement des filets internes (convoyage, malaxage et/ou contre-filet). Le fin contrôle des différents paramètres est opéré selon une pratique connue de l'Homme du métier. Les extrudeuses bis-vis peuvent être équipées d'une zone de lavage dans laquelle l'extrudat est dilué en continu par un flux d'eau de lavage pour permettre une séparation ultérieure plus aisée des fibres solides résiduelles et de la phase alcaline liquide contenant les polysaccharides et l'acide férulique salifié dissous. Il est encore avantageux d'équiper l'extrudeuse, spécialement l'extrudeuse bis-vis, d'un filtre fourreau pour réaliser la séparation solide/liquide subséquente *in situ.*

Les technologies utilisant des broyeurs homogénéiseurs sont également avantageuses pour dilacérer les fibres végétales et ainsi faciliter le contact avec la solution alcaline. Ils peuvent être mis en oeuvre en mode batch ou en mode continu. Par exemple, les broyeurs type Ultra Turrax® ou FRYMA® peuvent être utilisés à profit. Ces broyeurs peuvent être employés en étant immergés dans la solution alcaline ou être pilotés en ligne. On peut notamment coupler un broyeur en ligne et une cuve agitée pour avoir un temps de séjour suffisant (par exemple 2 à 8 heures de décoction alcaline) et une intensification du contact entre fibres végétale et solution alcaline.

Il est encore avantageux de coupler une technologie à fort coefficient de cisaillement avec une technologie utilisant des irradiations par micro-ondes ou des sondes à ultra-sons, lesquelles peuvent encore exacerber la libération d'acide férulique en accélérant localement les cinétiques d'hydrolyse.

Le traitement de ladite matière végétale selon ladite étape 1) du procédé de l'invention peut encore consister en un traitement enzymatique. Les enzymes opèrent une libération de l'acide férulique et des polysaccharides par hydrolyse des fonctions spécifiques, notamment des fonctions esters constitutives de la paroi végétale. Lesdites enzymes sont préférentiellement choisies parmi la cellulase, l'hémicellulase et la feruloyle esterase et leurs mélanges. Lesdites enzymes sont disponibles commercialement. Le traitement enzymatique est avantageusement opéré à une température comprise entre 20°C et 70°C pendant une durée comprise entre 0,5 et 20 heures. Il est avantageux de coupler la décoction alcaline décrite ci-dessus avec un traitement enzymatique pour maximiser la libération d'acide férulique (décoction alcalino-enzymatique). Il est encore avantageux de coupler le traitement enzymatique avec une mise en œuvre par extrusion (extrusion enzymatique).

De manière préférée, ledit traitement de matière végétale selon ladite étape 1) du procédé selon l'invention consiste en une décoction alcaline ou en une décoction alcalino-enzymatique.

L'étape de séparation solide/liquide associée à l'étape de traitement de matière végétale, préférentiellement à l'étape de décoction alcaline, est par exemple mise en oeuvre par centrifugation ou par filtration. La mise en oeuvre par centrifugation est avantageusement réalisée à l'aide d'une centrifugeuse à assiette ou décanteur centrifuge permettant une séparation en continu des phases solide et liquide. La phase solide récoltée à l'issue de l'étape de séparation solide/liquide contient des fibres de cellulose et d'hémicellulose constitutives des parois des cellules végétales. Cette phase solide peut avantageusement être valorisée dans diverses applications, notamment en nutrition animale. La phase liquide aqueuse obtenue à l'issue de ladite étape de séparation solide/liquide est composé principalement d'eau (au moins 90% poids), de sucres, notamment sous la forme de polysaccharides (avantageusement de 0,2 à 4% poids) et d'acide férulique (avantageusement de 10 à 10000 ppm). Ladite phase aqueuse est basique et présente un pH avantageusement supérieur à 9.

Conformément au procédé de l'invention, ladite phase liquide obtenue après séparation solide/liquide selon ladite étape 1) est soumise à une étape de séparation sélective des polysaccharides dissous dans ladite phase liquide (étape 2). Cette étape de séparation conduit à la production d'une fraction aqueuse constituée essentiellement de polysaccharides, d'une part, et d'une fraction aqueuse comprenant de l'acide férulique, d'autre part. La fraction constituée essentiellement de polysaccharides est telle que les polysaccharides représentent au moins 90% poids des composés de ladite fraction autres que l'eau. Ladite étape de séparation sélective est avantageusement mise en oeuvre par par adsorption sélective des polysaccharides ou par ultrafiltration, de manière très préférée par ultrafiltration. De manière préférée, ladite étape de séparation sélective des polysaccharides est mise en oeuvre par ultrafiltration au moyen d'une membrane organique ou inorganique, préférentiellement inorganique. Ladite membrane est par exemple de nature céramique ou polymérique. Ladite membrane utilisée pour la mise en oeuvre de ladite étape 2) d'ultrafiltration présente des pores dont le diamètre est compris entre 2 nm et 0,1 µm. Elle présente un seuil de coupure, défini comme étant la taille à partir de laquelle les molécules sont entièrement retenues par la membrane, compris entre 15000 g/mol et 300000 g/mol. Avantageusement, le seuil de coupure peut être compris entre 35000 g/mol et 300000 g/mol, de préférence entre 50000 g/mol et 300000 g/mol, de préférence entre 75000 g/mol et 300000 g/mol, et de manière encore plus préférée entre 100000 g/mol et 300000 g/mol. Le seuil de coupure de la membrane peut avantageusement être d'au moins 50000 g/mol.

Ladite étape d'ultrafiltration est avantageusement mise en oeuvre à une température inférieure à 100°C. Elle est préférentiellement mise en oeuvre à un pH compris entre 5 et 12.

Conformément à l'étape 2) du procédé selon l'invention, la membrane d'ultrafiltration retient les molécules organiques, en particulier les polysaccharides, de masse molaire supérieure à 15 000 g/mol présents dans la phase liquide issue de la séparation solide/liquide et laisse passer (perméat) une fraction aqueuse comprenant l'acide férulique sous forme de férulate ou d'un mélange acide férulique/férulate. Le rétentat est un flux aqueux dans lequel les composés (autres que l'eau) sont majoritairement les polysaccharides, lesquels représentent au moins 90% poids, de préférence au moins 95%, de l'ensemble des composés constituant le rétentat autres que l'eau. Au moins 90% poids, de préférence au moins 95% poids et de manière encore plus préférée au moins 98% poids, des polysaccharides présents initialement dans la phase liquide aqueuse issue de la séparation solide/liquide sont retenus par la membrane d'ultrafiltration. Lesdits polysaccharides présents dans le rétentat à l'issue de l'étape d'ultrafiltration sont avantageusement valorisés dans diverses applications, notamment en nutrition animale.

Ladite fraction aqueuse constituée essentiellement de polysaccharides obtenue à l'issue de ladite étape de séparation sélective, préférentiellement le rétentat de l'étape 2) d'ultrafiltration, est avantageusement concentré(e) par nanofiltration ou osmose inverse pour conduire à un rétentat plus riche en polysaccharides et un perméat constitué très majoritairement d'eau (> 99% poids) pouvant être recyclé à l'étape de traitement de matière végétale, préférentiellement de décoction alcaline.

Conformément à l'étape optionnelle 3) du procédé selon l'invention, la fraction aqueuse comprenant l'acide férulique et appauvrie en polysaccharides issue de l'étape de séparation sélective des polysaccharides, préférentiellement le perméat issu de ladite étape d'ultrafiltration, est soumis(e) à une étape de concentration (étape 3) de manière à produire un flux d'acide férulique concentré et un flux aqueux purifié. La concentration en acide férulique dans ledit flux concentré issu de l'étape de concentration est avantageusement au moins deux fois supérieure à celle de la fraction aqueuse issue de ladite étape 2), dans laquelle est présent l'acide férulique.

Ladite étape de concentration consiste avantageusement en la mise en oeuvre soit d'une étape d'évapo-concentration, soit d'une étape de séparation membranaire, notamment par nanofiltration ou par osmose inverse.

Selon un premier mode préféré de réalisation de ladite étape 3) du procédé selon l'invention, le perméat issu de ladite étape d'ultrafiltration est soumis à une étape de concentration par évapo-concentration mise en oeuvre au moyen d'un évaporateur à film raclé, d'un évaporateur à flot tombant ou d'un évaporateur triple effet fonctionnant de préférence à une pression inférieure à la pression atmosphérique. Le perméat issu de l'étape d'ultrafiltration se retrouve dans un concentrat dans lequel l'acide férulique est au moins 2 fois, voire 4 fois, plus concentré que dans ledit perméat et on récupère un distillat constitué d'eau pure pouvant être recyclé à l'étape de traitement de la matière végétale (étape 1), en particulier à l'étape de décoction alcaline.

Selon un deuxième mode préféré de réalisation de ladite étape 3) du procédé selon l'invention, le perméat issu de ladite étape d'ultrafiltration est soumis à une étape de nanofiltration mise en oeuvre au moyen d'une membrane organique ou inorganique, préférentiellement organique, dont le taux de réjection des sels (défini comme étant égal au pourcentage de soluté qui ne traverse pas la membrane) est au moins égal à 80%, de préférence au moins égal à 95%, préférentiellement au moins égal à 98%. Ladite membrane est une membrane commerciale. De manière plus préférée, ladite membrane utilisée pour la mise en oeuvre de ladite étape de nanofiltration est formée d'un ou plusieurs polymères, par exemple de polyamide, et présente un taux de réjection en MgSO₄ supérieur à 98%.

Selon un troisième mode préféré de réalisation de ladite étape 3) du procédé selon l'invention, le perméat issu de ladite étape d'ultrafiltration est soumis à une étape d'osmose inverse mise en oeuvre au moyen d'une membrane dont le diamètre des pores est compris entre 0,1 et 1 nm. On utilise une membrane organique ou inorganique, préférentiellement organique. Ladite membrane est une membrane commerciale. De manière plus préférée, ladite membrane utilisée pour la mise en oeuvre de ladite étape de nanofiltration est formée d'un ou plusieurs polymères, par exemple de polyamide, et présente un taux de réjection en MgSO₄ supérieur à 80%, de préférence supérieur à 98%, préférentiellement supérieur à 99%.

Ladite étape de séparation membranaire, par nanofiltration ou osmose inverse (2^{ème} et 3^{ème} modes décrits), est avantageusement mise en oeuvre à une température inférieure à 60°C. Elle est préférentiellement opérée à un pH inférieur à 9. Aussi, selon le pH auquel est opérée ladite étape 2) de séparation sélective des polysaccharides, le pH peut être à ajuster préalablement à ladite étape 3) de concentration.

Conformément aux deuxième et troisième modes préférés de réalisation de ladite étape 3) du procédé selon l'invention, la membrane réalisant la séparation membranaire laisse passer un flux d'eau purifiée (perméat) et retient un flux d'acide férulique concentré (rétentat) (sous forme de férulate ou d'un mélange d'acide férulique/férulate). Le perméat issu de ladite étape 3) est composé d'une eau purifiée dont la teneur en impuretés, notamment en polysaccharides et acide férulique est infinitésimale, c'est-à-dire moins de 0,1 % poids (basé sur l'exemple simulé), voire nulle. Le perméat à base d'eau purifiée est avantageusement recyclé à l'étape de traitement de la matière végétale produisant la phase liquide aqueuse de laquelle est extrait l'acide férulique selon le procédé de l'invention. Il en résulte ainsi une diminution notable de la consommation d'eau potable dans le procédé de traitement de la matière végétale, notamment de décoction alcaline de végétaux. Par flux d'acide férulique concentré, tel qu'obtenu dans le rétentat selon lesdits deuxième et troisième modes préférés de réalisation de ladite étape 3), il est entendu que la concentration en acide férulique et/ou férulate est au moins doublée à l'issue de la mise en oeuvre de ladite étape 3).

Selon le mode de mise en oeuvre du procédé de l'invention dans lequel l'étape 3) n'est pas opérée, la fraction aqueuse contenant l'acide férulique issue de ladite étape 2) est directement envoyée à l'étape de récupération sous forme solide cristallisée, préférentiellement en la soumettant préalablement à un traitement par évapo-concentration.

Conformément à l'étape 4) du procédé selon l'invention, on procède à un traitement dudit flux concentré en acide férulique formant le concentrat ou le rétentat issu de ladite étape 3) pour récupérer l'acide férulique sous forme solide cristallisée.

De manière préférée, ladite étape de récupération de l'acide férulique sous forme solide cristallisée est mise en oeuvre par cristallisation ou par atomisation. De manière très préférée, ladite étape de récupération selon ladite étape 4) du procédé selon l'invention est mise en oeuvre par cristallisation.

L'étape de récupération de l'acide férulique sous forme solide, de préférence l'étape de cristallisation, est préférentiellement précédée d'une étape de traitement dudit flux concentré en acide férulique consistant à procéder à l'acidification dudit flux concentré ou à une étape d'adsorption dudit flux concentré, l'acide férulique se présentant sous forme de férulate ou d'un mélange d'acide férulique/férulate.

S'agissant d'un traitement, préalable à la cristallisation, par acidification, tout acide minéral convient, en particulier l'acide sulfurique, l'acide phosphorique, l'acide chlorhydrique. L'acidification est effectuée de manière à obtenir un flux d'acide férulique ayant un pH inférieur à 7, préférentiellement inférieur à 5.

S'agissant d'un traitement, préalable à la cristallisation, par adsorption, ledit flux concentré en acide férulique issu de ladite étape 3) est mis en contact avec un matériau adsorbant capable d'adsorber sur sa surface l'acide férulique et/ou le férulate. Parmi les matériaux adsorbants avantageusement utilisés dans le procédé de l'invention, on peut citer notamment les charbons actifs et les résines polymères, échangeuses d'ions, adsorbantes ou d'exclusion stérique. Ledit matériau adsorbant est avantageusement placé soit dans un réacteur agité soit dans une colonne. Ladite étape d'adsorption est avantageusement mise en oeuvre à une température inférieure à 40°C. Lorsque l'adsorption est complète, le matériau adsorbant est récupéré par filtration ou régénéré par élution dans une colonne de manière à récupérer l'acide férulique. De manière préférée, on utilise un alcool, notamment l'éthanol, ou un sel, pour éluer l'acide férulique. La composition dudit flux concentré d'acide férulique oriente également le choix de l'éluant. Lorsque l'éluant est un sel et qu'en conséquence l'acide férulique se retrouve dans une fraction aqueuse, on procède à une acidification de ladite fraction aqueuse de manière à ce que l'acide férulique précipite. Lorsque l'éluant est un alcool, la(es) fraction(s) alcoolique(s) est (sont) évaporée(s) pour récupérer l'acide férulique sous forme solide. L'acide férulique ainsi récupéré présente une pureté insuffisante pour être utilisé ultérieurement comme matière première dans différents procédés nécessitant l'emploi de réactifs de haute pureté. Aussi l'acide férulique est soumis à une étape de recristallisation pour augmenter sa pureté. Ladite étape de recristallisation est avantageusement effectuée dans l'eau.

La cristallisation ou recristallisation de l'acide férulique conformément à l'étape 4) du procédé selon l'invention est opérée par refroidissement ou concentration du milieu dans lequel est présent l'acide férulique.

La cristallisation par refroidissement, à une température préférentiellement comprise entre 1°C et 10°C, conduit à la formation de cristaux d'acide férulique. Les cristaux d'acide férulique sont ensuite avantageusement filtrés, lavés puis séchés. La cristallisation est effectuée dans les appareillages classiquement utilisés tels que dans des réacteurs agités (appelés cristallisoirs) avec échangeurs internes et/ou circulation d'un fluide caloporteur dans une double enveloppe. Le séchage de l'acide férulique cristallisé est avantageusement conduit à une température comprise entre 50 et 100°C. Il est conduit selon des techniques bien connues de l'Homme du métier, par exemple en utilisant des séchoirs par contact à pression atmosphérique ou sous pression réduite, des séchoirs convectifs par l'air ou un gaz inerte. On peut également sécher l'acide férulique selon la technique du lit fluidisé. Les jus-mères et eaux de lavage, récoltés en fin de cristallisation, sont avantageusement envoyés vers une station d'épuration pour être traités et/ou purifiés.

La cristallisation par concentration consiste généralement à procéder à l'évaporation de l'eau dans laquelle est présent l'acide férulique. Cette étape d'évaporation est généralement opérée sous vide. Elle est mise en oeuvre selon des pratiques bien connues de l'Homme du métier.

A l'issue de la mise en oeuvre du procédé selon l'invention, l'acide férulique naturel obtenu présente une pureté élevée, généralement supérieure à 95% et de manière encore plus préférée supérieure à 99%.

L'acide férulique de haute pureté ainsi obtenu est avantageusement utilisé dans un procédé de bio-fermentation pour produire de la vanilline naturelle en présence de microorganismes, par exemple la souche *Streptomyces setonii.* La mise en oeuvre d'un tel procédé de production de vanilline naturelle à partir d'acide férulique et en présence d'une telle souche est décrit dans les brevets EP 0.7161.817 et EP 0.885.968 et conduit à un rendement en vanilline élevé. 18. L'invention a également pour objet un procédé de production de vanilline naturelle comprenant :
- l'extraction d'acide férulique présent dans une phase aqueuse, obtenue par traitement d'au moins une matière végétale, et contenant également des polysaccharides, tel que décrit précédemment, et
- la transformation de l'acide férulique ainsi obtenu en vanilline naturelle par un procédé de bio-fermentation en présence de microorganismes.

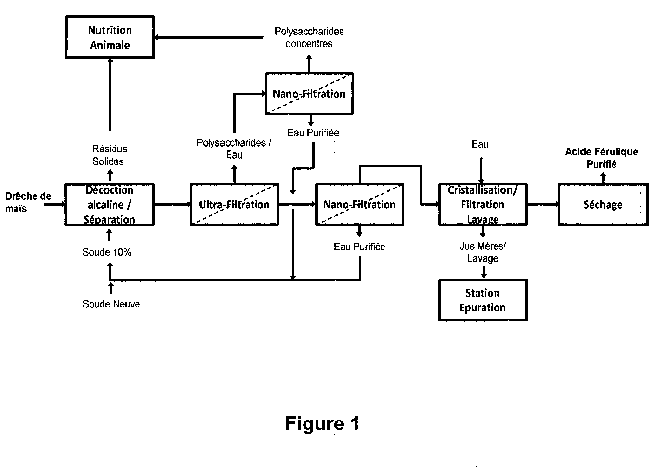

La figure 1 représente un enchaînement global intégrant les variantes préférées du procédé selon l'invention, notamment avec l'emploi de drêches de maïs pour la matière végétale à traiter, le traitement par décoction alcaline pour libérer l'acide férulique et les polysaccharides de la drêche de maïs, la séparation des polysaccharides par ultrafiltration puis la concentration de l'acide férulique par nanofiltration. Le schéma de la figure 1 montre également la purification du rétentat d'ultrafiltration par nanofiltration pour concentrer les polysaccharides, valorisés ultérieurement en alimentation animale. Ce schéma montre également qu'il est avantageux dans la mise en oeuvre du procédé selon l'invention de recycler les flux aqueux purifiés issus des étapes de nanofiltration vers l'étape de décoction alcaline.

L'invention est illustrée au moyen des exemples suivants.

### Exemple 1 (invention)

Des drêches de maïs (résidus d'amidonerie) sont introduites à l'alimentation d'une extrudeuse bi-vis (Battenfield-Cincinnati BEX2-28D) à un débit de 50 kg/h. La bi-vis de l'extrudeuse comporte six compartiments en plus de la section alimentation et est chauffée à une température de 100°C. Les six compartiments représentent un enchaînement d'éléments de convoyage, de malaxage, de contre-filets de pression et d'une zone de lavage en fin de bi-vis. Une solution de soude à 10% poids est introduite sur le deuxième compartiment de l'extrudeuse à un débit de 25 kg/h. Un flux d'eau de lavage est introduit sur le dernier compartiment à un débit de 400 kg/h. Le flux de décoction alcaline résultant, sortant de l'extrudeuse bi-vis, est récupéré dans un bac de stockage de 5 m³ où il est ramené à température ambiante.

Le flux aqueux de décoction alcaline est centrifugé en continu à l'aide une centrifugeuse à assiette de type Flottweg® pour séparer les matières solides résiduelles en suspension de la phase liquide contenant les polysaccharides et l'acide férulique dissous. On obtient ainsi une phase liquide aqueuse clarifiée contenant environ 600 ppm d'acide férulique sous forme de férulate de sodium et environ 5000 ppm de polysaccharides de haut poids moléculaire (supérieur à 3000 g/mole).

10 kg de phase liquide aqueuse clarifiée provenant de la décoction alcaline et contenant 6,1 g d'acide férulique et 49 g de sucres sous forme de polysaccharides sont prélevés. Le pH de ce flux est égal à pH=11,3. Le flux clarifié est amené à pH=7 par ajout d'acide sulfurique puis est traité sur une membrane commerciale céramique d'ultrafiltration (KERASEP® BX 300KD) présentant un support monolithique à base de TiO₂-Al₂O₃ et une couche active à base de ZrO₂-TiO₂ ayant un seuil de coupure de 300 000 g/mol, à une température de 50°C pour séparer les polysaccharides de masse supérieure à 300 000 g/mol qui restent dans le rétentat et l'acide férulique qui passe dans le perméat sous forme de férulate. On récolte 3 kg de rétentat contenant 48,5 g de sucres (polysaccharides) et 1,1 g d'acide férulique. On recueille 7 kg de perméat d'ultrafiltration contenant 5 g d'acide férulique. Le perméat est engagé, sur une membrane commerciale de nanofiltration Alfa Laval NF 99. La membrane de nanofiltration employée est une membrane spiralée en polyamide d'une surface de 0,34 m² avec un taux de réjection de MgSO₄ à 25°C d'au moins 98%. Elle présente un seuil de coupure compris entre 150 g/mol et 300 g/mol à une température de 50°C. Ce traitement permet d'obtenir un perméat correspondant à un flux d'eau purifiée: 5,75 kg contenant 130 ppm d'acide férulique, réutilisable directement à l'étape de décoction alcaline de la drêche de maïs et un rétentat correspondant à un flux d'acide férulique concentré : 1,25 kg contenant 4,25 g d'acide férulique.

Le rétentat récupéré à l'étape de nanofiltration est acidifié à pH=3 - 4 par ajout d'acide sulfurique puis refroidi à 2°C pour faire précipiter l'acide férulique. Le solide est filtré puis lavé 3 fois à l'eau pour donner après séchage 4 g d'acide férulique à 98% de pureté. Le rendement global de l'extraction d'acide férulique contenu dans la phase aqueuse clarifiée de décoction alcaline de la drêche de maïs est égal à 65,5%.

Le rétentat d'ultrafiltration contenant les polysaccharides peut également être concentré par nanofiltration pour donner un perméat correspondant à un flux d'eau purifiée contenant environ 100 ppm d'acide férulique qui peut être recyclé à l'étape de décoction alcaline et un rétentat correspondant à un flux de polysaccharides concentrés directement valorisable en nutrition animale ou en mélange avec les résidus solides séparés après l'étape de décoction alcaline.

## Revendications

1. Procédé d'extraction d'acide férulique présent dans une phase aqueuse, obtenue par traitement d'au moins une matière végétale, et contenant également des polysaccharides, ledit procédé comprenant au moins les étapes suivantes :
1) le traitement de ladite matière végétale suivi d'une séparation solide/liquide pour récupérer une phase solide et une phase liquide aqueuse comprenant l'acide férulique et lesdits polysaccharides,
2) le traitement de ladite phase liquide pour séparer sélectivement les polysaccharides d'une part et l'acide férulique présent dans une fraction aqueuse d'autre part, par adsorption sélective des polysaccharides ou par ultrafiltration,
3) éventuellement, la concentration de ladite fraction aqueuse contenant l'acide férulique pour récupérer un flux concentré en acide férulique,
4) la récupération sous forme solide de l'acide férulique.

2. Procédé selon la revendication 1 dans lequel ladite matière végétale est choisie parmi le son de blé, le son de riz, le son de maïs, le son d'avoine, les drêches de maïs, la bagasse, la pulpe de betterave et le résidu de raffinage de l'huile de son de riz et leurs mélanges.

3. Procédé selon la revendication 1 ou la revendication 2 dans lequel ledit traitement de ladite matière végétale consiste à soumettre la matière végétale à une décoction alcaline et/ou à un traitement enzymatique.

4. Procédé selon la revendication 3 dans lequel le traitement par décoction alcaline consiste à mettre en contact et cuire ladite matière végétale dans une solution alcaline ou une suspension alcaline.

5. Procédé selon l'une des revendications 1 à 4 dans lequel l'ultrafiltration est mise en oeuvre au moyen d'une membrane organique ou inorganique.

6. Procédé selon la revendication 5 dans lequel ladite membrane présente un seuil de coupure compris entre 50000 g/mol et 300000 g/mol.

7. Procédé selon l'une des revendications 5 à 6 dans lequel l'ultrafiltration est mise en oeuvre à un pH compris entre 5 et 12.

8. Procédé selon l'une des revendications 5 à 7 dans lequel au moins 90% poids des polysaccharides présents initialement dans la phase liquide aqueuse issue de la séparation solide/liquide sont retenus par la membrane d'ultrafiltration.

9. Procédé selon l'une des revendications 1 à 8 dans lequel ladite étape de concentration consiste en la mise en oeuvre soit d'une étape d'évapo-concentration soit d'une étape de séparation membranaire par nanofiltration ou par osmose inverse.

10. Procédé selon la revendication 9 dans lequel l'étape de séparation membranaire est opérée à un pH inférieur à 9.

11. Procédé selon la revendication 9 ou la revendication 10 dans lequel la concentration en acide férulique et/ou férulate est au moins doublée à l'issue de la mise en oeuvre de ladite étape 3).

12. Procédé selon l'une des revendications 1 à 11 dans lequel ladite étape de récupération selon ladite étape 4) est mise en oeuvre par cristallisation.

13. Procédé selon la revendication 12 dans lequel la cristallisation de l'acide férulique est précédée d'une étape de traitement dudit flux concentré en acide férulique consistant à procéder à l'acidification dudit flux concentré ou à une étape d'adsorption dudit flux concentré.

14. Procédé de production de vanilline naturelle comprenant :
- l'extraction d'acide férulique présent dans une phase aqueuse, obtenue par traitement d'au moins une matière végétale, et contenant également des polysaccharides, selon l'une des revendications 1 à 13, et
- la transformation de l'acide férulique ainsi obtenu en vanilline naturelle par un procédé de bio-fermentation en présence de microorganismes.

## Patentansprüche

1. Verfahren zur Extraktion von Ferulasäure, die in einer durch Behandlung von mindestens einem Pflanzenmaterial erhaltenen und auch Polysaccharide enthaltenden wässrigen Phase vorliegt, wobei das Verfahren mindestens die folgenden Schritte umfasst:
1) das Behandeln des Pflanzenmaterials mit nachfolgender Fest/Flüssig-Trennung zur Gewinnung einer festen Phase und einer wässrigen flüssigen Phase, die die Ferulasäure und die Polysaccharide enthält,
2) das Behandeln der flüssigen Phase zur selektiven Trennung der Polysaccharide einerseits und der in einer wässrigen Fraktion vorliegenden Ferulasäure andererseits durch selektive Adsorption der Polysaccharide oder durch Ultrafiltration,
3) gegebenenfalls das Aufkonzentrieren der die Ferulasäure enthaltenden wässrigen Fraktion zur Gewinnung eines konzentrierten Ferulasäurestroms,
4) das Gewinnen der Ferulasäure in fester Form.

2. Verfahren nach Anspruch 1, wobei das Pflanzenmaterial aus Weizenschrot, Reiskleie, Maiskleie, Haferkleie, Maistreber, Bagasse, Rübenpulpe und den Rückstand der Raffination von Reiskleieöl und Mischungen davon ausgewählt wird.

3. Verfahren nach Anspruch 1 oder Anspruch 2, wobei die Behandlung des Pflanzenmaterials daraus besteht, dass man das Pflanzenmaterial einem alkalischen Abkochen und/oder einer enzymatischen Behandlung unterwirft.

4. Verfahren nach Anspruch 3, wobei die Behandlung durch alkalisches Abkochen daraus besteht, dass man das Pflanzenmaterial mit einer alkalischen Lösung oder einer alkalischen Suspension in Kontakt bringt und darin kocht.

5. Verfahren nach einem der Ansprüche 1 bis 4, wobei die Ultrafiltration mit Hilfe einer organischen oder anorganischen Membran durchgeführt wird.

6. Verfahren nach Anspruch 5, wobei die Membran eine Ausschlussgrenze zwischen 50.000 g/mol und 300.000 g/mol aufweist.

7. Verfahren nach einem der Ansprüche 5 bis 6, wobei die Ultrafiltration bei einem pH-Wert zwischen 5 und 12 durchgeführt wird.

8. Verfahren nach einem der Ansprüche 5 bis 7, wobei mindestens 90 Gew.-% der anfänglich in der wässrigen flüssigen Phase aus der Fest/Flüssig-Trennung enthaltenen Polysaccharide durch die Ultrafiltration zurückgehalten werden.

9. Verfahren nach einem der Ansprüche 1 bis 8, wobei der Schritt des Aufkonzentrierens daraus besteht, dass man entweder einen Verdampfungsaufkonzentrierungsschritt oder einen Membrantrennungsschritt durch Nanofiltration oder Umkehrosmose durchführt.

10. Verfahren nach Anspruch 9, wobei der Membrantrennungsschritt bei einem pH-Wert von weniger als 9 durchgeführt wird.

11. Verfahren nach Anspruch 9 oder Anspruch 10, wobei die Konzentration von Ferulasäure und/oder Ferulat nach der Durchführung von Schritt 3) mindestens verdoppelt ist.

12. Verfahren nach einem der Ansprüche 1 bis 11, wobei der Gewinnungsschritt gemäß Schritt 4) durch Kristallisation durchgeführt wird.

13. Verfahren nach Anspruch 12, wobei der Kristallisation der Ferulasäure ein Schritt der Behandlung des konzentrierten Ferulasäurestroms vorgeschaltet ist, der aus dem Ansäuern des konzentrierten Stroms oder aus einem Schritt der Adsorption des konzentrierten Stroms besteht.

14. Verfahren zur Herstellung von natürlichem Vanillin, das Folgendes umfasst:
- Extrahieren von Ferulasäure, die in einer durch Behandlung von mindestens einem Pflanzenmaterial erhaltenen und auch Polysaccharide enthaltenden wässrigen Phase vorliegt, nach einem der Ansprüche 1 bis 13 und
- Umwandeln der so erhaltenen Ferulasäure in natürliches Vanillin durch ein Biofermentationsverfahren in Gegenwart von Mikroorganismen.

## Claims

1. Process for extraction of ferulic acid present in an aqueous phase obtained by treatment of at least one plant material, and also containing polysaccharides, said process comprising at least the following steps:
1) the treatment of said plant material followed by a solid/liquid separation to recover a solid phase and an aqueous liquid phase comprising the ferulic acid and said polysaccharides,
2) the treatment of said liquid phase to selectively separate, on the one hand, the polysaccharides and, on the other hand, the ferulic acid present in an aqueous fraction, by selective adsorption of the polysaccharides or by ultrafiltration,
3) optionally the concentration of said aqueous fraction containing the ferulic acid so as to recover a ferulic acid-concentrated stream,
4) the recovery of the ferulic acid in solid form.

2. Process according to Claim 1, wherein said plant material is selected from wheat bran, rice bran, corn bran, oat bran, spent corn grains, bagasse, beetroot pulp and the residue from refining rice bran oil, and mixtures thereof.

3. Process according to Claim 1 or Claim 2, wherein said treatment of said plant material consists in subjecting the plant material to an alkaline decoction and/or to an enzymatic treatment.

4. Process according to Claim 3, wherein the treatment by alkaline decoction consists in bringing said plant material into contact with and cooking it in an alkaline solution or an alkaline suspension.

5. Process according to one of Claims 1 to 4, wherein the ultrafiltration is carried out by means of an organic or inorganic membrane.

6. Process according to Claim 5, wherein said membrane has a cut-off threshold of between 50 000 g/mol and 300 000 g/mol.

7. Process according to either of Claims 5 and 6, wherein the ultrafiltration is carried out at a pH of between 5 and 12.

8. Process according to one of Claims 5 to 7, wherein at least 90% by weight of the polysaccharides initially present in the aqueous liquid phase resulting from the solid/liquid separation are retained by the ultrafiltration membrane.

9. Process according to one of Claims 1 to 8, wherein said concentration step consists in carrying out either an evapoconcentration step, or a membrane separation step by nanofiltration or by reverse osmosis.

10. Process according to Claim 9, wherein the membrane separation step is carried out at a pH of less than 9.

11. Process according to Claim 9 or Claim 10, wherein the concentration of ferulic acid and/or ferulate is at least doubled after said step 3) has been carried out.

12. Process according to one of Claims 1 to 11, wherein said recovery step according to step 4) is carried out by crystallization.

13. Process according to Claim 12, wherein the crystallization of the ferulic acid is preceded by a step of treating said ferulic acid-concentrated stream, consisting in acidifying said concentrated stream or consisting of a step of adsorption of said concentrated stream.

14. Process for producing natural vanillin, comprising:
- the extraction of ferulic acid present in an aqueous phase, obtained by treatment of at least one plant material, and also containing polysaccharides, according to one of Claims 1 to 13, and
- the conversion of the ferulic acid thus obtained into natural vanillin by means of a biofermentation process in the presence of microorganisms.
